# EUROPEAN PATENT SPECIFICATION

(11) **EP 1 592 649 B1**
(45) Date of publication and mention of the grant of the patent: **24.01.2007**
(21) Application number: 04707720.1
(22) Date of filing: 03.02.2004
(51) Int. Cl.: C07C 2/64, C07C 15/107

(54) **METHOD OF PREPARING BRANCHED ALKYL AROMATIC HYDROCARBONS USING A PROCESS STREAM FROM AN ISOMERIZATION UNIT**
METHODE ZUR HERSTELLUNG VON MIT VERZWEIGTEN ALKYLRESTEN SUBSTITUIERTEN AROMATISCHEN KOHLENWASERSTOFFEN UNTER VERWENDUNG EINES PROZESSSTROMES AUS EINER ISOMERISIERUNGSEINHEIT
PROCEDE DE PREPARATION D'HYDROCARBURES AROMATIQUES D'ALKYLE RAMIFIE METTANT EN OEUVRE UN FLUX DE TRAITEMENT EN PROVENANCE D'UNE UNITE D'ISOMERISATION

(30) Priority: 05.02.2003 US 445312 P
(43) Date of publication of application: 09.11.2005
(73) Proprietor: SHELL INTERNATIONALE RESEARCH MAATSCHAPPIJ B.V., 2596 HR Den Haag (NL)
(72) Inventor: AYOUB, Paul, Marie, NL-1031 CM Amsterdam (NL); DIRKZWAGER, Hendrik, NL-1031 CM Amsterdam (NL); MURRAY, Brendan, Dermot, Houston, TX 77077 (US); SUMROW, Steven, Clois, Katy, TX 77450 (US)
(86) International application number: PCT/US2004/002958
(87) International publication number: WO 2004/072000

(56) References cited:
- WO-A-02/44114
- US-A- 3 492 364

## Description

### BACKGROUND OF THE INVENTION

### 1. Field of Invention

The present invention generally relates to systems and methods for preparing alkyl aromatic hydrocarbons. More particularly, embodiments described herein relate to systems and methods for preparing branched alkyl aromatic hydrocarbons.

### 2. Description of Related Art

Alkylated aromatic hydrocarbons are important compounds that may be used in a variety of applications or converted to other chemical compounds (e.g. surfactants, sulfonates). Surfactants may be used in a variety of applications, for example detergents, soaps and oil recovery.

The structural composition of an alkyl aromatic hydrocarbon may influence the properties (e.g., water solubility, biodegradability, cold water detergency) of the surfactant and/or detergent produced from an alkyl aromatic hydrocarbon. For example, water solubility may be affected by linearity of the alkyl group. As the linearity of the alkyl group increases, the hydrophilicity (i.e., affinity for water) of the alkyl aromatic surfactant may decrease. Thus, the water solubility and/or detergency (performance) of the alkyl aromatic surfactant may decrease. Incorporating branches that contain a minimum number of quaternary and/or tertiary carbon atoms into the alkyl portion of the alkyl aromatic surfactant may increase the cold-water solubility and/or detergency of the alkyl aromatic surfactant while maintaining the biodegradability of a detergent. The amount and type of branching of the alkyl group, however, may decrease the biodegradation rate of a surfactant and/or detergent.

Branched alkyl aromatic hydrocarbons are composed of a branched alkyl group coupled to an aromatic group. Branched alkyl groups are composed of a linear alkyl groups with branches extending form the linear alkyl group. Branches of the linear alkyl groups may include one or more aliphatic alkyl groups, a linear alkyl group or combinations thereof. Branches may include methyl, ethyl, propyl or higher alkyl groups. Quaternary and tertiary carbons may be present in a branched the alkyl group. The number of quaternary and tertiary carbons may result from the branching pattern in the alkyl group. As used herein, the phrase "aliphatic quaternary carbon atom" refers to a carbon atom that is not bound to any hydrogen atoms and not attached to an aromatic ring system.

A surfactant with a branched alkyl group including quaternary and/or tertiary carbons may have a lower biodegradation rate than a surfactant with a linear or mono-branched alkyl group. As used herein, "biodegradable" refers to a material that can be chemically altered or broken down by bacteria or other natural agents. For example, in a biodegradation experiment using a porous pot activated sludge treatment, a biodegradation rate of sodium 2-methyl-2-undecyl[¹⁴C]benzensulfonate was greater than a biodegradation rate of sodium 5-methyl-5-undecyl[¹⁴C]benzensulfonate. A detailed description of the experiment is described by Nielsen et al. in "Biodegradation of Coproducts of Commercial Linear Alkylbenzene Sulfonate," Environmental Science and Technology, 1997, 31:3397-3404.

Examples of compositions and processes for the manufacture of branched alkyl aromatic hydrocarbons are described in U.S. Patent No. 3,484,498 to Berg, entitled "Process For The Preparation Of Aryl-Substituted Normal Paraffin Hydrocarbons"; U.S. Patent No. 6,111,158 to Marinangeli et al., entitled "Process For Producing Arylalkanes At Alk-ylation Conditions Using A Zeolite Having a NES Zeolite Structure Type" and U.S. Patent No. 6,187,981 to Marinangeli et al., entitled "Process For Producing Arylalkanes And Arylalkane Sulfonates, Compositions Produced Therefrom, and Uses Thereof".

More recently, it has been found that surfactants derived from branched olefins have different, more desirable properties than surfactants derived from linear olefins. For example, surfactants derived from branched olefins have increased water solubility, improved detergency properties and acceptable biodegradable properties compared to surfactants derived from linear olefins. Production of branched olefins from a Fischer-Tropsch process stream may increase an economic value of the stream. In some embodiments, linear olefins may be converted into branched olefins using an isomerization catalyst. The branched olefins may be used to produce surfactants that are more desirable and thus more valuable to the producer of the Fischer-Tropsch stream. In general, Fischer-Tropsch processes tend to produce minor amounts of olefins. Increasing an olefin content (e.g. branched alkyl olefin content) of a Fischer-Tropsch stream may increase the economic value of the stream. WO 02/44114 A1 discloses a method for the production of Alkyl-aryl-sulfonates.

### SUMMARY

In an embodiment, alkyl aromatic hydrocarbons may be produced by a method that includes isomerizing olefins in an isomerization unit Isomerized olefins may be used to alkylate aromatic hydrocarbons. After alkylation of the aromatic hydrocarbons, unreacted components from the alkylation process may be separated from the produced alkyl aromatic hydrocarbon products. Paraffins in the separated stream may be subjected to a dehydrogenation process to generate additional olefinic components. The resulting olefinic stream from the dehydrogenation process may be recycled back into the isomerization unit.

Isomerization of olefins in a process stream may occur in an isomerization unit. In an embodiment, a process feed stream entering an isomerization unit may include linear olefins and paraffins having an average carbon number from 7 to 16. In an embodiment, a process feed stream entering an isomerization unit includes linear olefins and paraffins having an average carbon number from 10 to 13. As used herein, the phrase "carbon number" refers to a total number of carbons in a molecule. In certain embodiments, a process feed stream entering an isomerization unit is derived from a Fischer-Tropsch process. In the isomerization unit, at least a portion of the linear olefins in a hydrocarbon stream may be isomerized to form branched olefins. The branched olefins may have an average number of branches per olefin molecule of between about 0.7 and about 2.5. A branched olefin may include, but is not limited to, methyl branching and/or ethyl branching. The isomerization process may produce branched olefins that include less than about 0.5 percent of aliphatic quaternary carbon atoms.

In an embodiment, one or more hydrocarbon streams may be combined with the feed stream entering the isomerization unit. The hydrocarbon stream may be mixed with the feed stream to alter the concentration of the olefins entering the isomerization unit. After the feed stream is processed in the isomerization unit, the resulting branched olefin containing stream may be passed into an alkylation unit. One or more hydrocarbon streams may be combined with the branched olefin containing stream to alter the concentration of olefins entering the alkylation unit.

Alkylation of aromatic hydrocarbons with olefins may occur in an alkylation unit. In an embodiment, an olefinic hydrocarbon stream from an isomerization unit and aromatic hydrocarbons may enter an alkylation unit. In the alkylation unit, at least a portion of the aromatic hydrocarbons may be alkylated with at least a portion of the olefins in the hydrocarbon stream to produce alkyl aromatic hydrocarbons. At least a portion of the produced alkyl aromatic hydrocarbons may include a branched alkyl group. At least a portion of the unreacted components of the hydrocarbon stream, at least a portion of unreacted aromatic hydrocarbons and at least a portion of the produced alkyl aromatic hydrocarbons may form an alkylation reaction stream.

At least a portion of the paraffins, unreacted olefins, aromatic hydrocarbons and alkyl aromatic hydrocarbons from the alkylation reaction stream may be separated to produce an unreacted hydrocarbon stream and an alkyl aromatic hydrocarbons product stream. The unreacted hydrocarbon stream may be further separated, in some embodiments, to form a paraffins and unreacted olefins stream and an aromatic hydrocarbon stream. At least a portion of the unreacted aromatic hydrocarbon stream may be recycled to the alkylation unit.

Dehydrogenation of paraffins may occur in a dehydrogenation unit. In an embodiment, at least a portion of a paraffins and unreacted olefins stream may enter a dehydrogenation unit. In the dehydrogenation unit, at least a portion of the paraffins in the paraffins and unreacted olefins stream may be dehydrogenated to produce olefins. At least a portion of the produced olefins may exit the dehydrogenation unit to from an olefinic hydrocarbon stream. The resulting olefinic hydrocarbon stream from the dehydrogenation process may be recycled back into the main process stream by sending the olefinic stream into the isomerization unit and/or into a stream entering the isomerization unit.

In certain embodiments, at least a portion of the alkyl aromatic hydrocarbon product streams from the above-described processes may be sulfonated to form alkyl aromatic sulfonates. In some embodiments, the alkyl aromatic sulfonates may include branched alkyl groups.

### BRIEF DESCRIPTION OF THE DRAWINGS

Advantages of the present invention will become apparent to those skilled in the art with the benefit of the following detailed description of embodiments and upon reference to the accompanying drawings, in which:

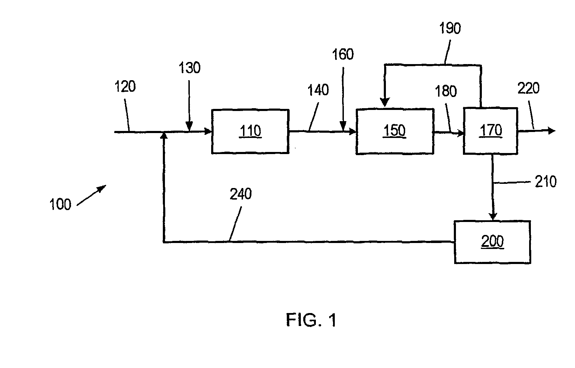

FIG. 1 depicts a schematic diagram of an embodiment of a system for producing branched alkyl aromatic hydrocarbons using an olefin isomerization unit.

While the invention is susceptible to various modifications and alternative forms, specific embodiments thereof are shown by way of example in the drawing and will herein be described in detail. It should be understood that the drawings and detailed description thereto are not intended to limit the invention to the particular form disclosed, but on the contrary, the intention is to cover all modifications, equivalents, and alternatives falling within the spirit and scope of the present invention as defined by the appended claims.

### DETAILED DESCRIPTION OF EMBODIMENTS

Hydrocarbon products may be synthesized from synthesis gas (i.e., a mixture of hydrogen and carbon monoxide) using a Fischer-Tropsch process. Synthesis gas may be derived from coal or by reforming of natural gas. The Fischer-Tropsch process catalytically converts synthesis gas into a mixture of products that includes primarily saturated hydrocarbons, a certain amount of olefins and a minor amount of oxygen containing products. The products from a Fischer-Tropsch process may be used for the production of fuels (e.g., gasoline, diesel oil), lubricating oils and waxes. Fuels and other products produced by a Fischer-Tropsch process generally have low levels of sulfur, nitrogen and/or metals and contain little or no cyclic compounds (e.g., (aromatics, naphthalenes).

Fischer-Tropsch process streams may also be used to prepare economically valuable commodity products. For example, linear olefins are commodity products that are useful for the production of surfactants. Using a portion of the process stream to produce linear olefins may increase the economic value of a Fischer-Tropsch process stream. To reduce production costs of producing alkyl aromatic hydrocarbons, a stream containing a significant amount of paraffins and a minor amount of olefins may first be isomerized, then alkylated to form alkyl aromatic hydrocarbons. Processing the low olefin feed stream through an isomerization unit prior to alkylation may save production time, catalyst cost, and/or enhance the overall economic viability of the low olefin process stream.

As described herein, a hydrocarbon feed stream composition may include paraffins and olefins. At least a portion of the hydrocarbon stream may be made up of linear paraffins and olefins having at least 4 carbon atoms and up to 16 carbon atoms. A hydrocarbon feed stream may be obtained from a Fisclier-Trogsch process and/or from an ethylene oligomerization process. Fischer-Tropsch catalysts and reaction conditions may be selected to provide a particular mix of products in the reaction product stream. Catalysts and/or combinations of catalyst that favor the manufacture of desired hydrocarbon species in a Fischer-Tropsch process are generally known.

While reference is made to a Fischer-Tropsch stream, it is to be understood that any stream, made by any process, that includes olefins and saturated hydrocarbons may be a suitable feedstock for the processes disclosed herein. Many Fischer-Tropsch streams may contain from 5 percent to 99 percent olefins, the remainder being saturated hydrocarbons and other compounds.

In some embodiments, feed streams containing olefms and paraffins are obtained through cracking of paraffin wax and/or the oligomerization of olefins. Commercial olefin products manufactured by ethylene oligomerization are marketed in the United States by Chevron Chemical Company, Shell Chemical Company (under the trademark NEODENE), and by British Petroleum. Cracking of paraffin wax to produce alpha-olefin and paraffin feed streams is described in U.S. Patent No. 4,579,986 to Sie, entitled "Process For The Preparation Of Hydrocarbons" and U.S. Patent Application Serial No. 10/153,955 to Ansorge et al., entitled "Process For The Preparation of linear Olefins and Use Thereof To Prepare Linear Alcohols". Specific procedures for preparing linear olefins from ethylene are described in U.S. Patent No. 3,676,523 to Mason, entitled "Alpha-Olefin Production"; U.S. Patent No. 3,686,351 to Mason, entitled "Alpha-Olefin Production; "U.S. Patent NO. 3,737,475 to Mason, entitled "Alpha-Olefin Production" and U.S. Patent No. 4,020,121 to Kister et al., entitled "Oligomerization Reaction System." Most of the above-mentioned processes produce alpha-olefins. Higher linear internal olefins may be commercially produced, for example, by the chlorination-dehydrochlorination of paraffins, by paraffin dehydrogenation, or by isomerization of alpha-olefins.

In an embodiment, a feed stream is processed to produce a hydrocarbon stream that includes branched olefins. Branched olefins may be used to alkylate an aromatic hydrocarbon to produce branched alkyl aromatic hydrocarbons. The feed stream may have a paraffin content range between about 50 percent by weight and about 90 percent by weight of the feed stream. In certain embodiments, a feed stream may have a paraffin content greater than about 90 percent by weight paraffins. The feed stream may also include olefins. The olefin content of the feed stream may be between about 10 percent by weight and about 50 percent by weight. In other embodiments, a feed stream may have an olefin content greater than 90 percent by weight olefins. The composition of the feed stream may include hydrocarbons having an average carbon number ranging from 4 to 30. In an embodiment, an average carbon number of the hydrocarbons in a feed stream may range from 4 to 24. In other embodiments, an average carbon number of a feed stream may range from 4 to 16, from 7 to 16, or from 10 to 13. A feed stream may include minor amounts of hydrocarbons having a carbon number that is higher and/or lower than the desired carbon number range. In some embodiments, a feed stream may be derived from distillation of a process stream that includes a broader range of carbon numbers.

In an embodiment, a feed stream for an isomerization unit includes mono-olefins and/or paraffins. The mono-olefins may be of a linear or branched structure. The mono-olefins may have an alpha or internal double bond. The feed stream may include olefins in which 50 percent or more of the olefin molecules present may be alpha-olefins of a linear (straight chain) carbon skeletal structure. In certain embodiments, at least about 70 percent of the olefins are alpha-olefins of a linear carbon skeletal structure. A hydrocarbon stream in which greater than about 70 percent of all of the olefin molecules are alpha-olefins of a linear carbon skeletal structure may be used in certain embodiments of alkylation of aromatic hydrocarbons. Such a stream may be derived from a Fischer-Tropsch process. In some embodiments, a feed stream includes olefins in which at least about 50 percent of the olefin molecules present are internal olefins.

Aromatic compounds (e.g., aromatic hydrocarbons) may include benzene or substituted benzene. In an embodiment, alkyl-substituted benzene is used as a substituted benzene in the process. Alkyl-substituted benzenes may be mono- and/or poly-substituted lower alkyl benzenes. The alkyl substituent of an alkyl substituted benzene may have a carbon number ranging from 1 to 5 or, in some embodiments, from 1 to 2. Suitable examples of aromatic compounds include, but are not limited to, benzene, toluene, xylenes, ethylbenzene, cumene, n-propylbenzene and other mono- and poly-lower alkylbenzenes. In some embodiments, a single aromatic compound and/or a mixture of two or more aromatic compounds may be used as a feed source. The aromatic hydrocarbons may be fed into the reactor directly or mixed in a suitable non-reactive organic solvent prior to addition to the alkylation unit.

System 100, in FIG. 1, depicts an embodiment of a method to produce branched alkyl aromatic hydrocarbons. A first hydrocarbon stream containing olefins and paraffins may enter isomerization unit 110 via first conduit 120. The first hydrocarbon stream may include hydrocarbons with an average carbon number from 7 to 16 or, in some embodiments, from 10 to 13. In some embodiments, a first hydrocarbon stream includes alpha-olefins. In certain embodiments, the first hydrocarbon stream is a stream derived from a Fischer-Tropsch process. The alpha-olefin content of the first hydrocarbon stream may be greater than about 70 percent of the total amount of olefins in the first hydrocarbon stream. In isomerization unit 110, at least a portion of the olefins in the first hydrocarbon stream may be isomerized to branched olefins to produce a second hydrocarbon stream.

In certain embodiments, isomerization unit 110 may have several points of entry to accommodate process streams of various composition. The process streams may be from other processing units and/or storage units. Examples of process streams include, but are not limited to, a diluent hydrocarbon stream and/or other hydrocarbon streams that include olefins and paraffins derived from other processes. As used herein "entry into the isomerization unit" is entry of process streams into the isomerization unit through one or more entry points.

The conditions for olefin isomerization in isomerization unit 110 may be controlled such that the number of carbon atoms in the olefins prior to and subsequent to the isomerization conditions is substantially the same. U. S. Patent No. 5,648,584 to Murray, entitled "Process for Isomerizing Linear Olefins to Isoolefins" and U.S. Patent No. 5,648,585 to Murray et al., entitled "Process for Isomerizing Linear Olefins to Isoolefins" describe catalysts and process conditions to skeletally isomerize linear olefins to branched olefins.

In an embodiment, linear olefins in a first hydrocarbon stream are isomerized in isomerization unit 110 by contacting at least a portion of the first hydrocarbon stream with a zeolite catalyst. The zeolite catalyst may have at least one channel with a crystallographic free channel diameter ranging from greater than 4.2 Å and less than about 7 Å. The zeolite catalyst may have an elliptical pore size large enough to permit entry of a linear olefin and diffusion, at least partially, of a branched olefin. The pore size of the zeolite catalyst may also be small enough to retard coke formation. As used herein, "coke" is thermal degradation product of a molecule into smaller molecules.

Temperatures at which the olefin isomerization may be conducted range from about 200 °C to about 500 °C. Temperatures in isomerization unit 110 are, in some embodiments, kept below the temperature at which the olefin will crack extensively. As used herein, "cracking" refers to the process of thermally degrading molecules into smaller molecules. To inhibit cracking, low temperatures may be used at low feed rates. In certain embodiments, lower temperatures may be used when an amount of oxygenates present in the process stream is low. Higher feed rates may be desirable to increase a production rate of isomerized products. Higher feed rates may be used, in some embodiments, when operating at higher reaction temperatures. The reaction temperature, however, should be set such that cracking to lower boiling weight products is minimized. For example, greater than 90 percent of linear olefins may be converted to branched olefins at 230 °C at a feed rate of 60 grams per hour with minimal cracking. Pressures maintained in isomerization unit 110 may be at a hydrocarbon partial pressure ranging from about 0.1 atmospheres (10 kPa) to about 20 atmospheres (2026 kPa). In an embodiment, a partial pressure may range from above about 0.5 atmospheres (51 kPa) to about 10 atmospheres (1013 kPa).

The branched olefins produced in isomerization unit 110 may include methyl, ethyl and/or longer carbon chain branches. Analysis of the isomerized olefin composition may be performed using Hydrogen Nuclear Magnetic Resonance (¹H NMR) techniques. Branched olefins may include quaternary and/or tertiary aliphatic carbons. In certain embodiments, an amount of quaternary and/or tertiary aliphatic carbons produced in an isomerization unit may be minimized. Analysis of olefins produced by the isomerization unit may indicate an extent of isomerization of olefins in a hydrocarbon stream.

The presence of quaternary carbon atoms may be determined using carbon 13 (¹³C) NMR techniques. The type of branching (e.g., methyl, ethyl, propyl or larger groups) may be determined by hydrogenation of an olefin mixture and then ¹³C NMR analysis of the hydrogenated olefin solution.

In an embodiment, an average number of branches per olefin molecule present in the branched olefin composition is between about 0.1 and about 2.5. In other embodiments, an average number of branches per olefin molecule present in the branched olefin composition is between about 0.7 and about 2.5. In certain embodiments, when the feed stream contains greater than 70 percent linear olefins, an average number of branches per olefin molecule present in the branched olefin composition is between about 0.2 and about 1.5. The degree of branching in the product may be controlled by controlling process conditions used in the isomerization unit. For example, high reaction temperatures and lower feed rates may result in a higher degree of branching. Methyl branches may represent between about 20 percent and about 99 percent of the total number of branches present in the olefin molecules. In some embodiments, methyl branches may represent greater than about 50 percent of the total number of branches in the olefin molecules. The number of ethyl branches in the olefin molecules may represent, in certain embodiments, less than about 30 percent of the total number of branches. In other embodiments, a number of ethyl branches, if present, may be between about 0.1 percent and about 2 percent of the total number of branches. Branches other than methyl or ethyl, if present, may be less than about 10 percent of the total number of branches.

The number of aliphatic quaternary and/or tertiary carbon atoms present in the branched olefin composition may be less than about 2 percent of the carbon atoms present. In an embodiment, an aliphatic quaternary and/or tertiary carbons present are less than about 1 percent of the carbon atoms present. For applications in which biodegradability is important, a number of aliphatic quaternary carbon atoms may be less than about 0.5 percent of the carbon atoms present. In an embodiment, a number of aliphatic quaternary and/or tertiary carbon atoms is less than about 0.3 percent of the carbon atoms present. In other embodiments, a number of aliphatic quaternary carbon atoms present in the branched olefin composition is from about 0.01 percent to about 0.3 percent of the aliphatic carbon atoms present.

In certain embodiments, additional hydrocarbon streams may be used to control reaction conditions and/or optimize a concentration of paraffins and unreacted olefins in isomerization unit 110. In an embodiment, at least a portion of a paraffinic hydrocarbon stream may be introduced into first conduit 120 via second conduit 130 upstream of isomerization unit 110 to produce a combined stream. The combined stream may enter isomerization unit 110 via first conduit 120. In other embodiments, a paraffinic hydrocarbon stream is introduced directly into isomerization unit 110 through one or more points of entry.

At least a portion of the olefins in the combined stream may be isomerized to branched olefins in isomerization unit 110 to produce a second hydrocarbon stream. Addition of a paraffinic hydrocarbon stream to isomerization unit 110 may be used to optimize the olefin concentration in isomerization unit 110 and to control the extent of branching in the produced olefins. The concentration of paraffins in the paraffinic hydrocarbon stream may be between about 10 percent and about 99 percent by weight. The paraffinic stream may also contain other hydrocarbons and/or olefins. In certain embodiments, a paraffin concentration may range between about 10 percent and about 50 percent by weight. In some embodiments, a paraffin concentration may range between about 25 percent and about 75 percent by weight.

Referring back to System 100, depicted in FIG. 1, isomerization unit 110 may produce a second hydrocarbon stream that includes olefins and paraffins. At least a portion of the second hydrocarbon stream contains branched olefins. The second hydrocarbon stream may exit isomerization unit 110 via third conduit 140 and be introduced into alkylation unit 150.

Alkylation of aromatic hydrocarbons by at least a portion of branched olefins produced in isomerization unit 110 may be conducted using various types of reactors. In certain embodiments, the process may be carried out in a batch wise fashion by adding the catalyst and aromatic hydrocarbons to a reactor, heating the mixture to a reaction temperature, and then adding the olefinic or aromatic hydrocarbons to the heated mixture. Control of the reaction temperature may be obtained by circulating a heat transfer fluid through the jacket of the reactor, evaporative cooling of the aromatic hydrocarbons or the use of a condenser. In some embodiments, a fixed bed reactor operating in an up flow or down flow mode or a moving bed reactor operating with co-current or countercurrent catalyst and hydrocarbon stream flows may be used. The fixed bed reactors may contain a single catalyst bed or multiple beds and may be equipped for the addition of olefins and cooling. The continuous removal of spent catalyst for regeneration and replacement by fresh or regenerated catalysts may be performed using a moving bed reactor. In other embodiments, a catalytic distillation reactor may also be used. Catalytic distillation reactors are described in U.S. Patent No. 6,291,719 to Gao et al., entitled "Methods And Equipments Of Using Dual Functional Catalyst Of Packing Type 2" and U.S. Patent No. 5,866, 736 to Chen, entitled "Process For Production Of Alkyl Benzene".

Alkylation unit 150 may have several points of entry to accommodate the entry of additional process streams. As used herein, "stream entering into the alkylation unit" is defined as the entry of process streams into the alkylation unit through one or more entry points. Examples of such process streams include, but are not limited to, streams from isomerization unit 110, a diluent hydrocarbon stream, gases and/or other hydrocarbon streams that include olefins and paraffins derived from other processes.

In an embodiment, at least a portion of the olefins in the second hydrocarbon stream, produced by isomerization unit 110, is contacted with aromatic hydrocarbons (e.g., benzene) in alkylation unit 150 using a variety of alkylating conditions. At least a portion of the resulting alkyl aromatic hydrocarbons are monoalkylated aromatic hydrocarbons having a branched alkyl group. Minimization of other alkylation products, such as dialkylation and higher alkylation products may be controlled by the process conditions (e.g., molar ratio, reaction temperatures, catalyst type and reactant concentrations) in alkylation unit 150.

In an embodiment, paraffins and other non-reactive components in the second hydrocarbon stream act as a diluent in the alkylation step. The presence of diluents in alkylation unit 150 may help control the temperature of the alkylation reaction. Diluents in alkylation unit 150 may also improve the selectivity of the alkylation process. Increased reaction temperatures in alkylation unit 150 may result in the formation of by-products (e.g., dialkylation and higher alkylation products).

The stoichiometry of the alkylation reaction requires only one mole of aromatic hydrocarbon compound per mole of total mono-olefins. Using 1:1 molar conditions, however, may result in mixtures of olefin oligomers and/or polymers, monoalkyl aromatic hydrocarbons, dialkyl-, trialkyl- and possibly highly polyalkylated aromatic hydrocarbons or unreacted olefins. A molar ratio as close to 1:1 as possible may maximize utilization of an aromatic hydrocarbon and minimize recycle of unreacted aromatic hydrocarbons or unreacted olefins. The molar proportion of aromatic hydrocarbon to total mono-olefin may influence both conversion and selectivity of the alkylation reaction. In certain embodiments, a molar ratio of total aromatic hydrocarbon per mole of total mono-olefins may be set between about 3:1 and about 100:1 to maximize formation of monoalkyl aromatic hydrocarbon. In some embodiments, an aromatic hydrocarbon to olefin ratio may be from about 5:1 to about 50:1. In other embodiments, an aromatic hydrocarbon to olefin ratio may be from about 5:1 to about 35:1. In certain embodiments, an aromatic hydrocarbon to total olefin ratio may be from about 8:1 to about 30:1.

In certain embodiments, additional hydrocarbon streams may be used to control reaction conditions and/or optimize a concentration of paraffins and unreacted olefins in alkylation unit 150. In certain embodiments, at least a portion of a third hydrocarbon stream may be introduced into third conduit 140 via fourth conduit 160 upstream of alkylation unit 150 to form a mixed stream. The mixed stream may be then introduced into alkylation unit 150 via third conduit 140. At least a portion of the olefins in the mixed stream may alkylate at least a portion of aromatic hydrocarbons. In some embodiments, a third hydrocarbon stream may be introduced directly into alkylation unit 150 through one or more points of entry. It should be understood that dilution of the process stream may be accomplished by adding a diluent stream through second conduit 130 only, fourth conduit 160 only, directly into alkylation unit 150 only or by a combination of points thereof.

The third hydrocarbon stream in fourth conduit 160 may be used to optimize the olefin concentration in alkylation unit 150 to maximize monoalkylation of the aromatic hydrocarbons and the amount of alkyl branching in the product. The third hydrocarbon stream may be from the same source as the first hydrocarbon stream. Alternatively, the third hydrocarbon stream may be a hydrocarbon stream that includes olefins, paraffins, and/or hydrocarbon solvents derived from another source. The third hydrocarbon stream may be derived from the same source as the paraffinic hydrocarbon stream introduced via second conduit 130.

The third hydrocarbon stream may include olefins and paraffins. In certain embodiments, an average carbon number of the hydrocarbons in the third hydrocarbon stream ranges from 7 to 16. The paraffin content of the third hydrocarbon stream may be between about 45 percent and about 99 percent by weight. In certain embodiments, a paraffin content of the third hydrocarbon stream may be between about 60 percent and about 90 percent by weight. In some embodiments, a paraffin content may be greater than about 80 percent by weight.

In an embodiment, an olefin content of a third hydrocarbon stream ranges between about 1 percent and about 99 percent relative to a total hydrocarbon content. In certain embodiments, an olefin content of a third hydrocarbon stream may be between about 5 percent and about 15 percent relative to the total hydrocarbon content. In other embodiments, an olefin concentration of the third hydrocarbon stream may be greater than about 80 percent by weight.

In some embodiments, the third hydrocarbon stream may include linear olefins. Addition of a stream that includes linear olefins downstream from the isomerization unit allows the creation of an alkylation feed stream that includes a mixture of linear and branched olefins. By introducing a stream including branched and linear olefins into alkylation unit 150, a mixture of branched and linear alkyl aromatic products may be obtained. Varying the amount of linear olefins added to the alkylation feed stream may control the ratio of linear to branched alkyl aromatic products. A mixture of branched and linear alkyl aromatic hydrocarbons may have improved properties when converted to alkyl aromatic surfactants. Examples of improved properties include, but are not limited to, low skin and eye irritation, foaming properties, biodegradability, cold-water solubility and cold-water detergency. Applications for these surfactants include, but are not limited to, personal care products, household and industrial laundry, hand dishwashing products, machine lubricant additives and lubricating oil formulations.

In an embodiment, alkylation conditions for alkylation of aromatic hydrocarbons in alkylation unit 150 may include the use of a Friedel-Crafts catalyst type. The Friedel-Crafts catalyst may be an acidic inorganic material. Examples of acidic inorganic materials include, but are not limited to, mineral acids such as sulfuric acid containing less than about 10 percent water, hydrofluoric acid containing less than about 10 percent water, liquefied anhydrous hydrogen fluoride, and/or other inorganic materials used in combination with hydrofluoric acid. Other inorganic materials may include, but are not limited to, Lewis acids such as anhydrous aluminum chloride, anhydrous aluminum bromide and boron trifluoride.

In certain embodiments, an alkylation catalyst may be a molecular sieve such as ITQ-21 in the acidic form. The synthesis and structures of molecular sieve catalysts are described by Corma, et al. in "A large-cavity zeolite with wide pore windows and potential as an oil refining catalyst," Nature, 2002, 418:514.

In certain embodiments, a catalyst used in alkylation unit 150 is based on a zeolite catalyst that may or may not be modified with a metal or metal compound. The zeolite catalyst may have at least one channel with a crystallographic free channel diameter ranging from greater than about 4 Å to less than about 9 Å with the understanding that when the pores have an elliptical shape, the larger pore size dimension is the dimension to be considered. In an embodiment, a pore size dimensions may range between about 5.5 Å to about 7 Å. A more detailed description of the catalyst may be found in U.S. Patent Application Serial No. 10/075,318 entitled "A Process For Preparing (Branched-Alkyl) Arylsulfonates and (Branched-Alkyl) Arylsulfonate Composition," U.S. Patent No. 6,111,158 to Marinangeli et al., entitled "Process For Producing Arylalkanes At Alkylation Conditions Using A Zeolite Having a NES Zeolite Structure Type" and U.S. Patent No. 5,041,402 to Schoennagel et al., entitled "Thermally Stable Noble Metal-Containing Zeolite Catalyst."

In alkylation unit 150, at least a portion of the olefins in the second hydrocarbon stream and at least a portion of the aromatic hydrocarbons may be reacted under alkylation conditions in the presence of the alkylation catalyst. Reaction temperatures for the alkylation reaction may range between greater than about 30 °C and less than about 300 °C. In certain embodiments, a reaction temperatures may range between greater than about 100°C and less than about 250°C. The alkylation reaction may be conducted in at least a partial liquid phase, in an all-liquid phase or at supercritical conditions. In certain embodiments, pressures in the alkylation unit are sufficient to maintain reactants in the liquid phase. The pressure used in alkylation unit 150 may depend upon the identity of olefin, the identity of the aromatic hydrocarbon and/or the temperature of the reaction. Pressures in alkylation unit 150 may range between about 3 atmospheres and about 70 atmospheres (304 kPa-7095 kPa). In certain embodiments, pressure may range between about 20 atmospheres and about 35 atmospheres (2025-3545 kPa).

Alkylation conditions in alkylation unit 150 may be maintained to minimize skeletal isomerization of the branched olefins. Alkylation conditions may also be maintained to produce alkyl aromatic hydrocarbons with an alkyl group that corresponds to the branching of the olefins produced in isomerization unit 110. "Skeletal isomerization during alkylation," as used herein, refers to isomerization during alkylation that changes the branching of an olefin or alkyl aromatic hydrocarbon product. Accordingly, alkylation conditions may be set such that a number of quaternary aliphatic carbon atoms in the olefin and the alkyl aromatic hydrocarbon product may remain unchanged during the alkylation reaction.

A general class of branched alkyl aromatic compounds produced in alkylation unit 150 may be characterized by the chemical formula R-Y, in which Y represents an aromatic hydrocarbyl radical (e.g., a phenyl radical) and R represents a radical derived from the olefin produced in isomerization unit 110. An olefin may have a carbon number in the range from 7 to 16. In certain embodiments, an olefin carbon number may range from 10 to 16. An olefin carbon number may, in other embodiments, range from 10 to 13. R may be a branched alkyl radical. Branches on a branched alkyl radical may include, but are not limited to, methyl, ethyl and/or longer carbon chains. An average number of branches per alkyl molecule present in the alkyl composition may be equal to the number of branches in the olefin produced in isomerization unit 110 (e.g., between 0.1 and 2.0).

The alkylation reaction mixture stream may enter separator 170 via fifth conduit 180. In separator 170 at least two streams, an unreacted hydrocarbon stream and an alkyl aromatic hydrocarbon stream may be produced. Separation of at least a portion of unreacted hydrocarbons from the produced branched alkyl aromatic hydrocarbons may be accomplished by methods known in the art (e.g., distillation, solid/liquid separation, adsorption, solvent extraction, etc.).

In certain embodiments, at least a portion of an alkylation reaction stream produced in alkylation unit 150 may pass through a solid/liquid separator (e.g., filter or centrifuge) to remove a solid catalyst and produce a catalyst-free alkylation stream. Next, at least a portion of the catalyst-free alkylation stream may pass through one or more distillation columns to produce an alkyl aromatic hydrocarbon stream, aromatic hydrocarbon stream, paraffins and unreacted olefins stream or combinations thereof. In certain embodiments, at least a portion of the aromatic hydrocarbon stream and at least a portion of the paraffins and unreacted olefins stream may be produced as one stream and further separated into an aromatic hydrocarbon stream and a paraffins and unreacted olefins stream using distillation techniques. At least a portion of the separated aromatic hydrocarbon stream may be recycled to alkylation unit 150 via sixth conduit 190. At least a portion of the separated paraffins and unreacted olefins may enter dehydrogenation unit 200 via seventh conduit 210. In other embodiments, after separation from the alkylation reaction mixture, at least a portion of the paraffins and unreacted olefins stream may be transferred to another processing unit and/or a storage vessel.

In certain embodiments, an average carbon number of the hydrocarbons in the paraffins and unreacted olefins stream may range from 7 to 16. In some embodiments, an average carbon number of the paraffins and unreacted olefins stream may range from 10 to 16. In other embodiments, an average carbon number of the hydrocarbons in the paraffins and unreacted olefins stream may range from 10 to 13.

In some embodiments, the alkyl aromatic hydrocarbon stream may contain unwanted higher molecular weight products. Passing at least a portion of the alkyl aromatic hydrocarbon stream through a distillation column to separate the alkyl aromatic hydrocarbon product from the heavier side products may further purify the alkyl aromatic hydrocarbon stream. At least a portion of the purified alkyl aromatic hydrocarbon product stream may be transferred through eighth conduit 220 to be stored on site, sold commercially, transported off-site and/or utilized in other processing units.

Referring back to System 100 depicted in FIG. 1, at least a portion of the paraffins and unreacted olefins stream may be introduced into dehydrogenation unit 200 via seventh conduit 210. At least a portion of the unreacted paraffins in the hydrocarbon stream may be dehydrogenated to produce an olefinic hydrocarbon stream by use of a catalyst selected from a wide range of catalyst types. For example, the catalyst may be based on a metal or metal compound deposited on a porous support. The metal or metal compound may be selected from, but is not limited to, chrome oxide, iron oxide and noble metals. "Noble metals" as used herein refers to metals of the group that includes platinum, palladium, iridium, ruthenium, osmium and rhodium.

Techniques of preparing catalysts, for performing the dehydrogenation step and for performing associated separation steps are known in the art. For example, suitable procedures for preparing catalysts and performing the dehydrogenation step are described in U.S. Patent No. 5,012,021 to Vora et al., entitled, "Process For the Production of Alkyl Aromatic Hydrocarbons Using Solid Catalysts;" U.S. Patent No. 3,274,287 to Moore et al., entitled, "Hydrocarbon Conversion Process and Catalyst;" U.S. Patent No. 3,315,007 to Abell et al., entitled, "Dehydrogenation of Saturated Hydrocarbons Over Noble-Metal Catalyst;" U. S. Patent No. 3,315,008 to Abell et al., entitled, "Dehydrogenation of Saturated Hydrocarbons Over Noble-Metal Catalyst," U.S. Patent No. 3,745,112 to Rausch, entitled, "Platinum-Tin Uniformly Dispersed Hydrocarbon Conversion Catalyst and Process;" U.S. Patent No. 4,506,032 to Imai et al., entitled, "Dehydrogenation Catalyst Composition" and U. S. Patent No. 4,430,517 to Imai et al., entitled, "Dehydrogenation Process Using a Catalytic Composition."

Reaction conditions in dehydrogenation unit 200 may be varied to control unwanted side products (e.g., coke, dienes, oligomers, cyclized hydrocarbons) and control double bond position in the olefin. In certain embodiments, temperatures may range from greater than about 300 °C to less than about 700 °C. In other embodiments, a reaction temperature may range from about 450 °C to about 550 °C. During the dehydrogenation reaction, pressures in dehydrogenation unit 200 may range from greater than about 1.0 atmosphere (101 kPa) to less than about 15 atmospheres (1520 LPa). In certain embodiments, pressure in dehydrogenation unit 200 may range from about 1.0 atmosphere (101 kPa) to about 5.0 atmospheres (510 kPa). To prevent coke from forming, hydrogen may be fed into dehydrogenation unit 200 together with the paraffins and unreacted olefins stream. The hydrogen to paraffins molar ratio may be set between about 0.1 moles of hydrogen to about 20 moles of paraffins. In some embodiments, a hydrogen to paraffin molar ratio is about 1 to 10.

The amount of time (e.g., the residence time) that a process stream remains in dehydrogenation unit 200 may determine, to some extent, an amount of olefins produced. Generally, the longer a process stream remains in dehydrogenation unit 200, a conversion level of paraffins to olefins increases until an olefin-paraffin thermodynamic equilibrium is obtained. The residence time of the paraffins and unreacted olefins stream in dehydrogenation unit 200 may be selected such that the conversion level of paraffins to olefins may be kept below 50 mole percent. In certain embodiments, a conversion level of paraffins to olefins may be kept in the range of from 5 to 30 mole percent. By keeping the conversion level low, side reactions (e.g., diene formation and cyclization reactions) may be inhibited.

Dehydrogenation unit 200 may receive at least a portion of the paraffins and unreacted olefins stream from separation unit 170 and produces an olefinic hydrocarbon stream. The olefinic hydrocarbon stream may include paraffins. The concentration of the olefins in the olefinic hydrocarbon stream may be between 5 and 50 percent by weight. In certain embodiments, a concentration of olefms may range between 10 and 20 percent by weight. The olefins produced in dehydrogenation unit 200 may be predominately linear olefins. The average carbon number of the hydrocarbons in the olefinic stream may range from 7 to 16. The average carbon number of the hydrocarbons in the olefinic stream ranges, in certain embodiments, from 10 to 16. In other embodiments, an average carbon number of the hydrocarbons in the olefinic stream may range from 10 to 13.

In certain embodiments, at least a portion of non-converted paraffins may be separated from the olefinic stream and, if desired, the non-converted paraffins may be recycled to dehydrogenation unit 200 to undergo further dehydrogenation. Such separation may be accomplished by extraction, distillation or adsorption techniques.

Referring back to System 100 as depicted in FIG. 1, the olefinic hydrocarbon stream may be combined with first hydrocarbon stream in first conduit 120 via ninth conduit 240. The combined stream may enter isomerization unit 110 and at least a portion of the olefins present in the combined stream may be isomerized to branched olefins. In some embodiments, an olefinic hydrocarbon stream may exit dehydrogenation unit 200 and be directly introduced into isomerization unit 110 through one or more points of entry.

The alkyl aromatic hydrocarbons product streams may be sulfonated in a sulfonation unit to form alkyl aromatic sulfonates. In certain embodiments, the alkyl aromatic hydrocarbon may contain branched alkyl groups. Sulfonation of at least a portion of the aromatic hydrocarbons in the alkyl aromatic hydrocarbon product stream may be performed by any method of sulfonation known in the art. Examples of such methods include sulfonation using sulfuric acid, chlorosulfonic acid, oleum or sulfur trioxide. Details of a sulfonation method involving an air/sulfur trioxide mixture are described in U.S. Patent No. 3,427,342 to Brooks et al., entitled "Continuous Sulfonation Process." In an embodiment, a sulfur trioxide to alkyl aromatic product molar ratio used for sulfonation is 1.03.

After the sulfonation reaction is complete, the sulfonation reaction mixture may be aged for about thirty minutes and then hydrolyzed with approximately 1% water. The resulting acid mixture may be neutralized with a base to produce a salt of the branched alkyl aromatic hydrocarbon sulfonate. Suitable neutralization bases may be hydroxides of alkali metals and alkaline earth metals, and ammonium hydroxides, which provide the cation M of the salts as described herein.

The general class of branched alkyl aromatic hydrocarbon sulfonates may be characterized by the chemical formula (R-A'-SO₃)*ₙ*M. R may represent a radical derived from the branched olefins, having an average carbon number in the range from 4 to 16. In an embodiment, an average carbon number ranges from 7 to 16. In another embodiment, an average carbon number ranges from 10 to 13. A' may represent a divalent aromatic hydrocarbyl radical, (e.g. a phenyl radical). M may be a cation selected from an alkali metal ion, an alkaline earth metal ion, an ammonium ion, and/or mixtures thereof and n may be a number depending on the valence of the cation(s) M, such that the total electrical charge of the complex is zero. In an embodiment, M may be sodium, magnesium or potassium ions. Magnesium and potassium ions may promote water solubility and performance of the alkyl aromatic hydrocarbon sulfonate. Examples of ammonium ions may include, but are not limited to, monoethanol amine, diethanol amine and triethanol amine. In certain embodiments, the ammonium ion may be represented by NH₄⁺. The resulting alkyl aromatic hydrocarbon sulfonate salt may be stored and/or sold as a solution (e.g. 30% aqueous solution), slurry (e.g., 60% aqueous slurry) and/or flakes (e.g., 90% dried flakes).

Branched alkyl aromatic sulfonates produced in the above-described processes may be used in a wide variety of applications. An example of an application includes detergent formulations. Detergent formulations include, but are not limited to, granular laundry detergent formulation, liquid laundry detergent formulations, liquid dishwashing detergent formulations and miscellaneous formulations. Examples of miscellaneous formulations include, but are not limited to, general purpose cleaning agents, liquid soaps, shampoos and liquid scouring agents.

### Examples

**Example 1: Isomerization of Olefins in a Fischer-Tropsch derived Hydrocarbon Stream:** Carbon monoxide and hydrogen were reacted under Fischer-Tropsch process conditions to yield a hydrocarbon mixture of linear paraffins, linear olefins, a minor amount of dienes and a minor amount of oxygenates. The Fischer-Tropsch hydrocarbon stream was separated into different hydrocarbon streams using fractional distillation techniques. A hydrocarbon stream containing olefins and paraffins with an average number of carbon atoms between 8 and 10 was obtained. The composition of the resulting C₈-C₁₀ hydrocarbon stream, tabulated in Table 1, was analysed by gas chromatography.

**Table 1**

| **Fischer-Tropsch Hydrocarbon Stream Composition** | **Wt%** |
|---|---|
| C₇ and lighter hydrocarbons | 0.12 |
| C₈ branched olefins | 0.02 |
| C₈ linear olefins | 0.75 |
| 1-Octene | 0.69 |
| n-Octane | 2.21 |
| C₉ branched olefins | 0.16 |
| C₉ linear olefins | 8.52 |
| 1-Nonene | 8.07 |
| n-Nonane | 20.03 |
| C₁₀ branched olefins | 0.28 |
| C₁₀ linear olefins | 22.92 |
| 1-Decene | 20.87 |
| n-Decane | 41.12 |
| C₁₁ and heavier hydrocarbons | 0.21 |
| C₉-C₁₁ alcohols | 3.56 |

A zeolite catalyst used for isomerization of linear olefins in the hydrocarbon stream was prepared in the following manner. Ammonium-ferrierite (645 grams) exhibiting a 5.4% loss on ignition and exhibiting the following properties: molar silica to alumina ratio of 62:1, surface area of 369 square meters per gram (P/Po = 0.03), soda content of 480 ppm and n-hexane sorption capacity of 7.3 g per 100 g of ammonium-ferrierite was loaded into a Lancaster mix muller. CATAPAL® D alumina (91 grams) exhibiting a loss on ignition of 25.7% was added to the muller. During a five-minute mulling period, 152 milliliters of deionized water was added to the alumina/ammonium-ferrierite mixture. Next, a mixture of 6.8 grams glacial acetic acid, 7.0 grams of citric acid and 152 milliliters of deionized water was slowly added to the alumina/ammonium-ferrierite mixture in the muller to peptize the alumina. The resulting alumina/ammonium-ferrierite/acid mixture was mulled for 10 minutes. Over a period of 15 minutes, a mixture of 0.20 grams of tetraamine palladium nitrate in 153 grams of deionized water was slowly added to mulled alumina/ammonium-ferrierite/acid mixture. The resulting mixture exhibited a 90:10 ratio of zeolite to alumina and a loss on ignition of 43.5%. The zeolite/alumina mixture was shaped by extruding the mixture through a stainless steel die plate (1/16" holes) of a 2.25 inch Bonnot extruder.

The moist zeolite/alumina extrudate was dried at 125 °C for 16 hours. After drying, the zeolite/alumina extrudate was longsbroken manually. The zeolite/alumina extrudate was calcined in flowing air at 200 °C for two hours. The temperature was raised to a maximum temperature of 500 °C and the zeolite/alumina extrudate was calcined for an additional two hours to yield an isomerization catalyst. The isomerization catalyst was allowed to cool in a dessicator under a nitrogen atmosphere.

Stainless steel tubing, 1 inch OD, 0.6 inch ID and 26 inches long, was used as an isomerization reactor. A thermowell extended 20 inches from the top of the stainless steel reactor tube. To load the reactor tube, the reactor tube was inverted and a piece of glass wool was transferred down the wall of the reactor tube, over the thermowell and positioned at the bottom of the reactor tube to serve as a plug for the reactor tube. Silicon carbide (20 mesh) was added to a depth of about 6 inches to the reactor tube. A second piece of glass wool was placed over the silicon carbide. A mixture of 6.0 grams of the isomerization catalyst particles (6-20 mesh) and 45 grams of fresh silicon carbide (60-80 mesh) was added to the reactor tube in two parts. The two-part addition distributed the isomerization catalyst evenly in the reactor tube and resulted in an isomerization catalyst bed of about 10 inches in length. A third piece of glass wool was added to the top of the catalyst in the reactor tube. Silicon carbide (20 mesh) was layered onto the third piece of glass wool. A fourth piece of glass wool was positioned over the silicon carbide to serve as a plug for the bottom of the reactor tube. To monitor the temperature of the reaction at various points in the reactor tube, a multipoint thermocouple was inserted into the thermowell of the reactor tube. The temperature above, below and at three different places in the catalyst bed was monitored. The reactor tube was inverted and installed in the furnace. The reactor tube was heated to an operating temperature of 280°C over a four-hour period under flowing nitrogen. Once the temperature of 280 °C was obtained, the reactor tube was held at the operating temperature for an additional two hours to condition the isomerization catalyst.

After conditioning the isomerization catalyst, the hydrocarbon stream was pumped through the reactor tube at a flow rate of 60 g/hr. Nitrogen, at a flow rate of 6 L/hr, was passed over the isomerization catalyst simultaneously with the hydrocarbon stream. The hydrocarbon stream was vaporized before contacting the isomerization catalyst. The reactor tube was operated at an outlet pressure of 20 kPa above atmospheric pressure.

In Table 2, the weight percent of C₈-C₁₀ branched olefins, C₈-C₁₀ linear olefins and C₈-C₁₀ paraffins in the hydrocarbon stream at 0 hours and in the reactor tube effluent after 24 and 48 hours of isomerization is tabulated. Greater than 90% of the linear olefins in the hydrocarbon stream were converted into branched olefins in the isomerization reactor. During the isomerization step, a small amount of material boiling below C₈ was generated from cracking side reactions. In addition, a portion of the C₉-C₁₁ alcohols present in the feed was dehydrated to yield additional olefins in the product. The average number of alkyl branches on the C₈-C₁₀ olefins in the product was found to be 1.0 as determined by the ¹H NMR techniques.

**Table 2**

| **Fischer-Tropsch Hydrocarbon Stream Composition During Isomerization Reaction** | **0 Hr Wt%** | **24 Hr Wt%** | **48 Hr Wt%** |
|---|---|---|---|
| C₈-C₁₀ branched olefins | 0.46 | 33.04 | 33.16 |
| C₈-C₁₀ linear olefins | 32.19 | 2.52 | 2.54 |
| C₈-C₁₀ paraffins | 63.19 | 63.32 | 63.27 |
| Branched to linear C₈₋₁₀ olefin ratio | 0.1 | 13.1 | 13.1 |

**Example 2. Isomerization of 1-Dodecene:** 1-Dodecene was obtained from Shell Chemical Co. The composition of 1-dodecene, as assayed by gas chromatography, is tabulated in Table 3.

**Table 3**

| **1-Dodecene Composition** | **Wt %** |
|---|---|
| 1-Dodecene | 98.0 |
| Other C₁₀-C₁₄ olefins | 1.2 |
| <C₁₀ hydrocarbons | 0.2 |
| >C₁₄ hydrocarbons | 0.2 |
| Paraffins | 0.4 |
| Total C₁₀-C₁₄ hydrocarbons | 99.6 |

1-Dodecene was isomerized using the same reactor tube design and isomerization catalyst preparation as described in Example 1. A stream of 1-dodecene was pumped through a reactor tube at a flow rate of 90 g/hr. Nitrogen, at a flow rate of 6 L/hr, was passed over the isomerization catalyst simultaneously with the stream of 1-dodecene. The stream of 1-dodecene was vaporised before contacting the isomerization catalyst. The reactor tube was operated at an outlet pressure of 20 kPa above atmospheric pressure and a temperature of 290°C.

Table 4 is a tabulation of the weight percent of hydrocarbons with carbon numbers less than C₁₀, from C₁₀-C₁₄ and greater than C₁₄ molecules in 1-dodecene at 0 hours and the reactor tube effluent after 168 and 849 hours. Linear C₁₀-C₁₄ olefins were converted in a 94% yield to branched C₁₀-C₁₄ olefins after a 168 hr processing time. During the isomerization step less than 3 weight percent of material boiling below C₁₀ was generated from cracking side reactions. The average number of alkyl branches on the C₁₀-C₁₄ olefins in the product was determined to be 1.3 by the ¹H NMR techniques.

**Table 4**

| **1-Dodecene Stream Composition During Isomerization Reaction** | **0 Hr Wt%** | **168 Hr Wt%** | **849 Hr Wt%** |
|---|---|---|---|
| <C ₁₀ hydrocarbons | 0.2 | 2.5 | 2.4 |
| C₁₀-C₁₄ hydrocarbons | 99.6 | 97.2 | 97.4 |
| >C₁₄ hydrocarbons | 0.2 | 0.3 | 0.2 |
| Branched C₁₀-C₁₄ olefins | 0.6 | 93.2 | 93.4 |
| Linear C₁₀-C₁₄ olefins | 99.0 | 2.8 | 2.9 |
| Paraffins | 1.0 | 2.0 | 1.9 |

## Claims

1. A method for the production of alkyl aromatic hydrocarbons, comprising:
introducing a first hydrocarbon stream comprising olefins and paraffins into an isomerization unit, wherein the isomerization unit is configured to isomerize at least a portion of linear olefins in the first hydrocarbon stream to branched olefins, and wherein at least a portion of the unreacted components of the first hydrocarbon stream and at least a portion of the produced branched olefins form a second hydrocarbon stream;
introducing at least a portion of the second hydrocarbon stream and aromatic hydrocarbons into an alkylation unit, wherein the alkylation unit is configured to alkylate at least a portion of the aromatic hydrocarbons with at least a portion of the olefins in the second hydrocarbon stream to produce alkyl aromatic hydrocarbons, wherein at least a portion of the produced alkyl aromatic hydrocarbons comprise a branched alkyl group, and wherein at least a portion of the unreacted components of the second hydrocarbon stream, at least a portion of the aromatic hydrocarbons and at least a portion of the produced alkyl aromatic hydrocarbons form an alkylation reaction stream;
separating alkyl aromatic hydrocarbons from the alkylation reaction stream to produce an unreacted hydrocarbon stream and an alkyl aromatic hydrocarbon stream, the unreacted hydrocarbon stream comprising at least a portion of the unreacted components of the second hydrocarbon stream and aromatic hydrocarbons;
separating at least a portion of the paraffins and at least a portion of the olefins from the unreacted hydrocarbon stream to produce an aromatic hydrocarbon stream and a paraffins and unreacted olefins stream; and
introducing at least a portion of the paraffins and unreacted olefins stream into a dehydrogenation unit, wherein the dehydrogenation unit is configured to dehydrogenate at least a portion of paraffins in the paraffins and unreacted olefins stream to produce olefins, and wherein at least a portion of the produced olefins exit the dehydrogenation unit to form an olefinic hydrocarbon stream; and
introducing at least a portion of the olefinic hydrocarbon stream into the isomerization unit.

2. The method of claim 1, wherein the first hydrocarbon stream is produced from an olefin oligomerization process or from a Fischer-Tropsch process.

3. The method of any one of claims 1 to 2, wherein the first hydrocarbon stream comprises olefins and paraffins having a carbon number from 10 to 13 or a carbon number from 10 to 16.

4. The method of any one of claims 1 to 3, wherein the isomerization unit is operated at a reaction temperature between about 200 °C and about 500 °C.

5. The method of any one of claims 1 to 4, wherein the isomerization unit is operated at a reaction pressure between about 0.1 atmospheres and about 10 atmospheres.

6. The method of any one of claims 1 to 5, wherein at least a portion of the branched olefins comprise methyl and ethyl branches.

7. The method of any one of claims 1 to 5, wherein a portion of the branched olefins comprise an average number of branches per total olefin molecules of at least 0.7, between about 0.7 and about 1.5, between about 0.7 and about 2.0, between about 1.0 and about 1.5, or less than about 2.5.

8. The method of any one of claims 1 to 7, wherein the branched groups on the branched olefins are greater than about 50 percent methyl groups, less than about 10 percent ethyl groups, or less than about 5 percent groups neither methyl or ethyl groups.

9. The method of any one of claims 1 to 8, wherein the branched olefins have less than about 0.5 percent aliphatic quaternary carbon atoms or less than about 0.3 percent aliphatic quaternary carbon atoms.

10. The method of any one of claims 1 to 9, wherein the alkylation unit is configured to produce greater than about 50 percent or greater than about 85 percent of monoalkylated aromatic hydrocarbons.

11. The method of any one of claims 1 to 10, wherein a molar ratio of the aromatic hydrocarbons to the branched olefins is between about 0.1 and about 2.0 in the alkylation unit

12. The method of any one of claims 1 to 11, wherein the alkylation unit is operated at a reaction temperature between about 30 °C and about 300 °C.

13. The method of any one of claims 1 to 12, wherein the aromatic hydrocarbons comprise benzene.

14. The method of any one of claims 1 to 12, wherein the aromatic hydrocarbons comprise alkylbenzenes.

15. The method of any one of claims 1 to 14, further comprising adjusting a ratio of olefins to paraffins introduced into the isomerization unit by adding at least a portion of a paraffinic hydrocarbon stream into the isomerization unit; or by combining a paraffinic hydrocarbon stream with at least a portion of the first hydrocarbon stream upstream of the isomerization unit to form a combined stream and introducing the combined stream into the isomerization unit.

16. The method of any one of claims 1 to 14, further comprising adjusting a ratio of olefins to paraffins introduced into the alkylation unit by adding at least a portion of a third hydrocarbon stream into the alkylation unit; or by adding at least a portion of a third hydrocarbon stream into the alkylation unit, wherein the third hydrocarbon stream comprises greater than about 90 percent paraffins by weight.

17. The method of any one of claims 1 to 14, further comprising:
adjusting a ratio of olefins to paraffins introduced into the alkylation unit by combining at least a portion of a third hydrocarbon stream with at least a portion of the second hydrocarbon stream upstream of the alkylation unit to form a combined stream; by combining at least a portion of a third hydrocarbon stream with at least a portion of the second hydrocarbon stream upstream of the alkylation unit to form a combined stream, wherein the third hydrocarbon stream comprises greater than about 80, greater than about 90, or between 85 and 95 percent paraffins by weight; by combining at least a portion of a third hydrocarbon stream with at least a portion of the second hydrocarbon stream upstream of the alkylation unit to form a combined stream, wherein the third hydrocarbon stream comprises paraffins and olefins having a carbon number from 10 to 16; by combining at least a portion of a third hydrocarbon stream with at least a portion of the second hydrocarbon stream upstream of the alkylation unit to form a combined stream, wherein the third hydrocarbon stream comprises linear olefins; by combining at least a portion of a third hydrocarbon stream with at least a portion of the second hydrocarbon stream upstream of the alkylation unit to form a combined stream, wherein at least a portion of the second hydrocarbon stream comprises branched olefins; by combining at least a portion of a third hydrocarbon stream with at least a portion of the second hydrocarbon stream upstream of the alkylation unit to form a combined stream, wherein at least a portion of the third hydrocarbon stream comprises linear olefins and at least a portion of the second hydrocarbon stream comprises branched olefins; and
introducing the combined stream into the alkylation unit.

18. The method of any one of claims 1 to 14, further comprising:
adjusting a ratio of olefins to paraffins introduced into the isomerization unit by combining at least a portion of a paraffinic hydrocarbon stream with at least a portion of the first hydrocarbon stream upstream of the isomerization unit to form a combined stream;
introducing the combined stream into the isomerization unit;
adjusting a ratio of olefins to paraffins introduced into the alkylation unit by combining at least a portion of a third hydrocarbon stream with at least a portion of the second hydrocarbon stream upstream of the alkylation unit to form a combined stream; and
introducing the combined stream into the alkylation unit

19. The method of any one of claims 1 to 18, wherein the dehydrogenation unit is operated at a temperature between about 300 °C and about 700 °C.

20. The method of any one of claims 1 to 19, wherein the dehydrogenation unit is operated at a pressure between about 1.0 atmosphere and about 15 atmospheres.

21. The method of any one of claims 1 to 20, wherein a residence time of at least a portion of the unreacted hydrocarbon stream in the dehydrogenation unit is such that the conversion level of the paraffins in the unreacted hydrocarbon stream composition to olefins is less than about 50 mole percent.

22. The method any one of claims 1 to 21, wherein introducing the olefinic hydrocarbon stream into the isomerization unit comprises combining at least a portion of the olefinic hydrocarbon stream with at least a portion of the first hydrocarbon stream to produce a combined stream upstream from the isomerization unit, and introducing at least a portion of the combined stream into the isomerization unit

23. The method of any one of claims 1 to 22, further comprising separating non-converted paraffins from the olefinic stream and introducing at least a portion of the non-converted paraffins separated from the olefinic stream into the dehydrogenation unit.

24. The method of any one of claims 1 to 23, further comprising introducing at least a portion of the alkyl aromatic hydrocarbon stream into a sulfonation unit, wherein the sulfonation unit is configured to sulfonate at least a portion of the alkyl aromatic hydrocarbons in the alkyl aromatic hydrocarbon stream to produce alkyl aromatic sulfonates, wherein at least a portion of the alkyl aromatic sulfonates produced comprise branched alkyl aromatic sulfonates.

## Patentansprüche

1. Eine Methode zur Erzeugung von alkylaromatischen Kohlenwasserstoffen, beinhaltend:
das Einführen eines ersten Stromes von Kohlenwasserstoffen zusammengesetzt aus Olefinen und Paraffinen in eine Isomerisierungseinheit, wobei die Isomerisierungseinheit gestaltet ist, um wenigstens einen Teil der unverzweigten Olefine des ersten Kohlenwasserstroms in verzweigte Olefine zu isomerisieren, und wobei wenigstens ein Teil der nicht umgesetzten Komponenten des ersten Stroms von Kohlenwasserstoffen und wenigstens ein Teil der erzeugten verzweigten Olefine einen zweiten Strom von Kohlenwasserstoffen bildet;
das Einführen von wenigstens einem Teil des zweiten Stroms von Kohlenwasserstoffen und aromatischen Kohlenwasserstoffen in eine Alkylierungseinheit, wobei die Alkylierungseinheit zur Alkylierung von wenigstens einem Teil der aromatischen Kohlenwasserstoffen mit wenigstens einem Teil der Olefine des zweiten Kohlenwasserstroms zu alkylaromatischen Kohlenwasserstoffe gestaltet ist, wobei wenigstens ein Teil der erzeugten alkylaromatischen Kohlenwasserstoffe eine verzweigte Alkylgruppe umfasst,
und wobei wenigstens ein Teil der nicht umgesetzten Komponenten des zweiten Stroms von Kohlenwasserstoffen, wenigstens ein Teil der aromatischen Kohlenwasserstoffe und wenigstens einen Teil der erzeugten alkylaromatischen Kohlenwasserstoffe einen Alkylierungsreaktionsstrom bilden;
das Abtrennen der alkylaromatischen Kohlenwasserstoffe vom Alkylierungsreaktionsstrom, um so einen Strom von nicht umgesetzten Kohlenwasserstoffen und einen Strom von alkylaromatischen Kohlenwasserstoffen zu erzeugen, wobei der Strom von nicht umgesetzten Kohlenwasserstoffen wenigstens einen Teil der nicht umgesetzten Kohlenwasserstoffe des zweiten Stroms von Kohlenwasserstoffen und aromatischen Kohlenwasserstoffen umfasst;
das Abtrennen von wenigstens einem Teil der Parafine und wenigstens einem Teil der Olefine vom Strom der nicht umgesetzten Kohlenwasserstoffe, um so einen Strom von aromatischen Kohlenwasserstoffen und einen Strom von Paraffinen und nicht umgesetzten Olefinen zu erzeugen;
das Einführen wenigstens eines Teils des Stroms von Paraffinen und nicht umgesetzter Olefine in eine Dehydrogenierungseinheit zur Dehydrogenierung von wenigstens einem Teil der Paraffine des Stroms der Paraffine und nicht umgesetzter Olefine um Olefine zu erzeugen, wobei wenigstens ein Teil der erzeugten Olefinen die Dehydrogenierungseinheit verlässt, um einen olefinischen Kohlenwasserstoffstrom zu bilden;
und das Einführen wenigstens eines Teil des olefinischen Kohlenwasserstoffstroms in die Isomerisierungseinheit.

2. Die Methode von Anspruch 1, wobei der erste Strom von Kohlenwasserstoffen von einem Olefin-Oligomerisationsverfahren oder einem Fischer-Tropschverfahren ausgeht.

3. Die Methode einer der Ansprüche von 1 oder 2, wobei der erste Kohlenwasserstoffstrom Olefine und Paraffine umfasst mit einer Kohlenstoffzahl zwischen 10 und 13 oder zwischen 10 und 16.

4. Die Methode einer der Ansprüche von 1 bis 3, bei der die Isomerisierungseinheit bei einer Reaktionstemperatur zwischen ungefähr 200°C und ungefähr 500°C betrieben wird.

5. Die Methode einer der Ansprüche von 1 bis 4, bei der die Isomerisierungseinheit bei einem Reaktionsdruck zwischen ungefähr 0,1 Atmosphären und ungefähr 10 Atmosphären betrieben wird.

6. Die Methode einer der Ansprüche von 1 bis 5, bei der wenigstens ein Teil der verzweigten Olefine Methyl - und Ethylverzweigungen enthält.

7. Die Methode einer der Ansprüche von 1 bis 5 bei der ein Teil der verzweigten Olefine eine Durchschnittszahl von Verzweigungen pro Gesamtolefinmolekül von mindestens 0.7, zwischen ungefähr 0.7 und ungefähr 1.5, zwischen ungefähr 0.7 und ungefähr 2.0, zwischen ungefähr 1.0 und ungefähr 1.5, oder weniger als ungefähr 2.5 enthält.

8. Die Methode einer der Ansprüche von 1 bis 7, wobei die verzweigten Gruppen der verzweigten Olefine zu mehr als ungefähr 50% aus Methylgruppen, zu weniger als ungefähr 10% aus Ethylgruppen, oder zu weniger als ungefähr 5% aus Gruppen die weder Ethyl noch Methylgruppen sind, bestehen.

9. Die Methode einer der Ansprüche von 1 bis 8, wobei die verzweigten Olefine weniger als ungefähr 0,5% aliphatischer quaternärer Kohlenstoffatome oder weniger als ungefähr 3% aliphatischer quaternärer Kohlenstoffatome beinhalten.

10. Die Methode einer der Ansprüche von 1 bis 9, wobei die Alkylierungseinheit so gestaltet ist, um mehr als ungefähr 50% oder mehr als ungefähr 85% aromatischer Monoalkylkohlenwasserstoffe zu erzeugen.

11. Die Methode einer der Ansprüche von 1 bis 10, wobei in der Alkylierungseinheit das Molverhältnis zwischen den aromatischen Kohlenwasserstoffen und den verzweigten Olefinen zwischen ungefähr 0.1 und ungefähr 2.0 liegt.

12. Die Methode einer de Ansprüche von 1 bis 11, wobei die Alkylierungseinheit bei einer Reaktionstemperatur betreiben wird, die zwischen ungefähr 30 °C und ungefähr 300 °C liegt.

13. Die Methode einer der Ansprüche von 1 bis 12, bei welcher die aromatischen Kohlenwasserstoffe Benzol beinhalten.

14. Die Methode einer der Ansprüche von 1 bis 12, wobei die aromatischen Kohlenwasserstoffe Alkylbenzole beinhalten.

15. Die Methode einer der Ansprüche von 1 bis 14, ausserdem umfassend das Angleichen des Verhältnisses zwischen Olefinen und Paraffinen die in die Isomerisierungseinheit eingeführt werden, durch Zufügen wenigstens eines Teiles von einem Strom paraffinischer Kohlwasserstoffe zur Isomerisierungseinheit; oder durch kombinieren eines Stromes paraffinischer Kohlwasserstoffe mit wenigstens einem Teil des ersten Stroms von Kohlwasserstoffen vor Eintreten in die Isomerisierungseinheit zu einem kombinierten Strom, und das Einbringen des kombinierten Stromes in die Isomerisierungseinheit.

16. Die Methode einer der Ansprüche von 1 bis 14, die ausserdem das Verhältnis von Olefinen zu Paraffinen, welche in die Alkylierungseinheit eingeführt werden, angleicht, durch Einbringen wenigstens eines Teils eines dritten Kohlenwasserstroms in die Alkylierungseinheit; oder durch Einbringen wenigstens eines Teils eines dritten Kohlenwasserstroms in die Alkylierungseinheit, wobei der dritte Strom von Kohlenwasserstoffen ungefähr 90% an Gewicht an Paraffinen umfasst.

17. Die Methode einer der Ansprüche von 1 bis 14, die ausserdem das Angleichen des Verhältnisses von Olefinen zu Paraffinen, welche in die Alkylierungseinheit eingeführt werden, umfasst, durch Zusammenfügen wenigstens eines Teils eines dritten Stroms von Kohlenwasserstoffen mit wenigstens einem Teil eines zweiten Stroms von Kohlenwasserstoffen, wobei der dritte Kohlenwasserstoffstrom mehr als ungefähr 80, mehr als ungefähr 90 oder zwischen 85 und 95% Gewicht an Paraffinen umfasst;
durch Zusammenfügen von wenigstens einem Teil eines dritten Kohlenwasserstoffstroms mit wenigstens einem Teil eines zweiten Kohlenwasserstoffstroms vor Eintritt in die Alkylierungseinheit unter Bildung eines zusammengeführten Stroms, wobei von wenigstens einem Teil des dritten Kohlenwasserstoffstroms Paraffine und Olefine umfasst die 10 bis 16 Kohlenstoffe enthalten;
durch Zusammenfügen von wenigstens einem Teil eines dritten Kohlenwasserstoffstroms mit wenigstens einem Teil eines zweiten Kohlenwasserstoffstroms vor Eintritt in die Alkylierungseinheit unter Bildung eines zusammengeführten Stroms, wobei wenigstens ein Teil des dritten Kohlenwasserstoffstrom unverzweigte Olefine enthält;
durch Zusammenfügen von wenigstens einem Teil eines dritten Kohlenwasserstoffstroms mit wenigstens einem Teil eines zweiten Kohlenwasserstoffstroms vor Eintritt in die Alkylierungseinheit unter Bildung eines zusammengeführten Stroms, wobei wenigstens ein Teil des zweiten Kohlenwasserstoffstroms verzweigte Olefine enthält;
durch Zusammenfügen von wenigstens einem Teil eines dritten Kohlenwasserstoffstroms mit wenigstens einem Teil eines zweiten Kohlenwasserstoffstroms vor Eintritt in die Alkylierungseinheit unter Bildung eines zusammengeführten Stroms, wobei wenigstens ein Teil des dritten Kohlenwasserstoffstrom unverzweigte Olefine enthält und wobei wenigstens ein Teil des zweiten Kohlenwasserstoffstroms verzweigte Olefine enthält;
und das Eingbringen der zusammengefügten Stroms in die Alkylierungseinheit.

18. Die Methode einer der Ansprüche von 1 bis 14, die ausserdem das Angleichen des Verhältnisses von Olefinen zu Paraffinen, welche in die Isomerisierungseinheit eingeführt werden, umfasst, wobei wenigstens ein Teil des paraffinischen Kohlenwasserstoffstroms mit wenigstens einem Teil des ersten Kohlenwasserstoffstroms vor Eintritt in die Isomerisierungseinheit zusammengefügt wird;
Einführen des kombinierten Stroms in die Isomerisierungseinheit; und
Angleichen des Verhältnisses von Olefinen zu Paraffinen, welche in die Alkylierungseinheit eingeführt werden durch Zusammenfügen von wenigstens eines Teils des dritten Kohlenwasserstoffstroms mit zumindest einem Teil des zweiten Kohlenwasserstoffstroms vor Einbringen in die Alkylierungseinheit, und
Einbringen des so erhaltenen zusammengefügten Stroms in die Alkylierungseinheit.

19. Die Methode einer der Ansprüche von 1 bis 18, wobei die Dehydrogenierungseinheit bei einer Temperatur von zwischen ungefähr 300 °C und ungefähr 700 °C betrieben wird.

20. Die Methode einer der Ansprüche von 1 bis 19, wobei die Dehydrogenierungseinheit bei einem Druck zwischen ungefähr 1.0 Atmosphären und ungefähr 15 Atmosphären betrieben wird.

21. Die Methode einer der Ansprüche von 1 bis 20, wobei die Verbleibzeit wenigstens eines Teils des nicht umgesetzten Kohlenwasserstoffsstroms in der Dehydrogenierungseinheit so ist, dass der Umsatz der Paraffine in Zusammensetzung des nicht umgesetzten Kohlenwasserstoffsstroms in Olefine unter ungefähr 50 Mol % liegt.

22. Die Methode einer der Ansprüche von 1 bis 21, wobei das Einbringen des olefinischen Kohlenwasserstoffstroms in die Isomerisierungseinheit das Zusammenfügen von wenigstens einem Teil des olefinischen Kohlenwasserstoffstroms mit wenigstens einem Teil des ersten Kohlenwasserstoffstroms enthält, um einen zusammengeführten Strom zu erzeugen, und Einführen wenigstens einen Teil des zusammengeführten Stroms in die Isomerisierungseinheit.

23. Die Methode einer der Ansprüche von 1 bis 22, die außerdem die Abtrennung der nicht umgesetzten Paraffine vom olefinischen Strom, und die Einführung von wenigstens einem Teil der nicht umgesetzten und vom olefinischen Strom abgetrennten Paraffinen in die Dehydrogenierungseinheit umfasst.

24. Die Methode einer der Ansprüche von 1 bis 23, die außerdem die Einführung von wenigstens einem Teil des alkylaromatischen Kohlenwasserstoffstroms in eine Sulfonierungsseinheit umfasst, wobei die Sulfonierungsseinheit zur Sulfonierung von wenigstens einem Teil der alkylaromatischen Kohlenwasserstoffe ausgestattet ist, um alkylaromatische Sulfonate zu erzeugen, wobei zumindest ein Teil der alkylaromatischen Sulfonate verzweigte alkylaromatischen Sulfonate umfassen.

## Revendications

1. Une méthode pour produire des hydrocarbures alkyl aromatiques qui comprend:
introduire un premier courant d'hydrocarbures composé d'oléfines et de paraffines dans une unité d'isomérisation, où l'unité d'isomérisation est configurée pour isomériser au moins une partie des oléfines linéaires du premier courant d'hydrocarbures en oléfines ramifiées, et où au moins une partie des composants non--réagi du premier courant d'hydrocarbures et au moins une partie des oléfines ramifiées produites forment un second courant d'hydrocarbures;
introduire au moins une partie du second courant d'hydrocarbures et hydrocarbures aromatiques dans une unité d'alkylation, où l'unité d'alkylation est configurée pour alkyler au moins une partie des hydrocarbures aromatiques avec au moins une partie des oléfines du second courant d'hydrocarbures, de manière de produire d'hydrocarbures alkyl aromatiques, où au moins une partie des hydrocarbures alkyl aromatiques produits comprennent un groupe alkyl ramifié, et où au moins une partie des composants non-réagi du second courant d'hydrocarbures, au moins une partie des hydrocarbures aromatiques et au moins une partie des hydrocarbures alkyl aromatiques produits, forment un courant pour une réaction d'alkylation;
séparer les hydrocarbures alkyl aromatiques du courant pour la réaction d'alkylation de façon de produire un courant d'hydrocarbures non-réagi et un courant d'hydrocarbures alkyl aromatiques, comprenant le courant d'hydrocarbures non-réagi, au moins une partie des composants non-réagi du second courant d'hydrocarbures et hydrocarbures aromatiques;
séparer au moins une partie des paraffines et au moins une partie des oléfines du courant d'hydrocarbures non-réagi, de sorte de produire un courant d'hydrocarbures aromatiques et un courant de paraffines et oléfines non-réagi; et
introduire au moins une partie du courant de paraffines et oléfines non-réagi dans une unité de déshydrogénation, où l'unité de déshydrogénation est configurée pour déshydrogéner au moins une partie des paraffines du courant de paraffines et oléfines non-réagi, de façon de produire des oléfines, et où au moins une partie des oléfines produites sortent de l'unité de déshydrogénation pour former un courant d'hydrocarbures oléfiniques; et
introduire au moins une partie du courant d'hydrocarbures oléfiniques dans l'unité d'isomérisation.

2. La méthode de la revendication 1, où le premier courant d'hydrocarbures se produit à partir d'un processus d'oligomérisation d'oléfines ou d'un processus de Fischer-Tropsch.

3. La méthode de n'importe quelles des revendications 1 à 2, où le premier courant d'hydrocarbures comprend des oléfines et des paraffines ayant un nombre de carbones de 10 à 13 ou un nombre de carbones de 10 à 16.

4. La méthode de n'importe quelles des revendications 1 à 3, où l'unité d'isomérisation est opérée à une température de réaction comprise entre environ 200 °C et environ 500 °C.

5. La méthode de n'importe quelles des revendications 1 à 4, où l'unité d'isomérisation est opérée à une pression de réaction comprise entre environ 0.1 atmosphères et environ 10 atmosphères.

6. La méthode de n'importe quelles des revendications 1 à 5, où au moins une partie des oléfines ramifiées comprend des branches méthyle et éthyle.

7. La méthode de n'importe quelles des revendications 1 à 5, où une partie des oléfines ramifiées comprend un nombre moyen de branches par molécules totales d'oléfine d'au moins 0.7, entre environ 0.7 et environ 1.5, entre environ 0.7 et environ 2.0, entre environ 1.0 et environ 1.5, ou moins d'environ 2.5.

8. La méthode de n'importe quelles des revendications 1 à 7, où les groupes ramifiés des oléfines ramifiées consistent plus d'environ 50 pour cent en groupes méthyle, moins d'environ 10 pour cent en groupes éthyle, ou moins d'environ 5 pour cent en groupes n'étant ni méthyle, ni éthyle.

9. La méthode de n'importe quelles des revendications 1 à 8, où les oléfines ramifiées ont moins d'environ 0.5 pour cent d'atomes de carbone quaternaires aliphatiques ou moins d'environ 0.3 pour cent d'atomes de carbone quaternaires aliphatiques.

10. La méthode de n'importe quelles des revendications 1 à 9, où l'unité d'alkylation est configurée pour produire plus d'environ 50 pour cent ou plus d'environ 85 pour cent d'hydrocarbures aromatiques monoalkylés.

11. La méthode de n'importe quelles des revendications 1 à 10, où dans l'unité d'alkylation la relation molaire entre les hydrocarbures aromatiques et les oléfines ramifiées est comprise entre environ 0.1 et environ 2.0.

12. La méthode de n'importe quelles des revendications 1 à 11, où l'unité d'alkylation est opérée à une température de réaction comprise entre approximativement 30 °C et environ 300 °C.

13. La méthode de n'importe quelles des revendications 1 à 12, où les hydrocarbures aromatiques comprennent du benzène.

14. La méthode de n'importe quelles des revendications 1 à 12, où les hydrocarbures aromatiques comprennent alkyl benzènes.

15. La méthode de n'importe quelles des revendications 1 à 14, que comprend en outre ajuster la proportion d'oléfines et de paraffines qu'on introduit dans l'unité d'isomérisation, en ajoutant au moins une partie d'un courant d'hydrocarbures paraffiniques dans l'unité d'isomérisation; ou bien en combinant un courant d'hydrocarbures paraffiniques avec au moins une partie du premier courant d'hydrocarbures en amont de l'unité d'isomérisation, de sorte de former un courant combiné et d'introduire le courant combiné dans l'unité d'isomérisation.

16. La méthode de n'importe quelles des revendications 1 à 14, qui comprend en outre ajuster la proportion d'oléfines et de paraffines qu'on introduit dans l'unité d'alkylation, en ajoutant au moins une partie d'un troisième courant d'hydrocarbures dans l'unité d'alkylation; ou bien en ajoutant au moins une partie d'un troisième courant d'hydrocarbures dans l'unité d'alkylation, où le troisième courant d'hydrocarbures comprend plus d'environ 90 pour cent en poids de paraffines.

17. La méthode de n'importe quelles des revendications 1 à 14, qui comprend en plus:
ajuster la proportion d'oléfines et de paraffines qui sont introduites dans l'unité d'alkylation, en combinant au moins une partie d'un troisième courant d'hydrocarbures avec au moins une partie du second courant d'hydrocarbures en amont de l'unité d'alkylation, de manière de former un courant combiné; en combinant au moins une partie d'un troisième courant d'hydrocarbures avec au moins une partie du second courant d'hydrocarbures en amont de l'unité d'alkylation, de façon de former un courant combiné, où le troisième courant d'hydrocarbures comprend plus d'environ 80, plus d'environ 90, ou entre 85 et 95 pour cent en poids de paraffines; en combinant au moins une partie d'un troisième courant d'hydrocarbures avec au moins une partie du second courant d'hydrocarbures en amont de l'unité d'alkylation, de manière de former un courant combiné, où le troisième courant d'hydrocarbures comprend des paraffines et des oléfines ayant un nombre de carbones de 10 à 16; en combinant au moins une partie d'un troisième courant d'hydrocarbures avec au moins une partie du second courant d'hydrocarbures en amont de l'unité d'alkylation, de manière de former un courant combiné, où le troisième courant d'hydrocarbures comprend des oléfines linéaires; en combinant au moins une partie du troisième courant d'hydrocarbures avec au moins une partie du second courant d'hydrocarbures en amont de l'unité d'alkylation, de façon de former un courant combiné, où au moins une partie du second courant d'hydrocarbures comprend des oléfines ramifiées; en combinant au moins une partie d'un troisième courant d'hydrocarbures avec au moins une partie du second courant d'hydrocarbures en amont de l'unité d'alkylation, de manière de former un courant combiné, où au moins une partie du troisième courant d'hydrocarbures comprend des oléfines linéaires et au moins une partie du second courant d'hydrocarbures comprend des oléfines ramifiées, et
introduire le courant combiné dans l'unité d'alkylation.

18. La méthode de n'importe quelles des revendications 1 à 14, qui comprend en outre:
ajuster la proportion d'oléfines et de paraffines qui sont introduites dans l'unité d'isomérisation, en combinant au moins une partie d'un courant d'hydrocarbures paraffiniques avec au moins une partie du premier courant d'hydrocarbures en amont de l'unité d'isomérisation, de manière de former un courant combiné;
introduire le courant combiné dans l'unité d'isomérisation;
ajuster la proportion d'oléfines et de paraffines qui sont introduites dans l'unité d'alkylation en combinant au moins une partie d'un troisième courant d'hydrocarbures avec au moins une partie du second courant d'hydrocarbures en amont de l'unité d'alkylation, de manière de former un courant combiné; et
introduire le courant combiné dans l'unité d'alkylation.

19. La méthode de n'importe quelles des revendications 1 à 18, où l'unité de déshydrogénation est opérée à une température comprise entre environ 300°C et environ 700°C.

20. La méthode de n'importe quelles des revendications 1 à 19, où l'unité de déshydrogénation est opérée à une pression comprise entre environ 1.0 atmosphère et environ 15 atmosphères.

21. La méthode de n'importe quelles des revendications 1 à 20, où le temps de permanence d'au moins une partie du courant d'hydrocarbures non-réagi dans l'unité de déshydrogénation est tel que le niveau de conversion des paraffines présentes dans la composition du courant d'hydrocarbures non--réagi dans des oléfines, est inférieur à environ 50 pour cent en moles.

22. La méthode de n'importe quelles des revendications 1 à 21, où introduire le courant d'hydrocarbures oléfiniques dans l'unité d'isomérisation comprend combiner au moins une partie du courant d'hydrocarbures oléfiniques avec au moins une partie du premier courant d'hydrocarbures, de façon de produire un courant combiné en amont de l'unité d'isomérisation, et d'introduire au moins une partie du courant combiné dans l'unité d'isomérisation.

23. La méthode de n'importe quelles des revendications 1 à 22, qui comprend en outre séparer les paraffines non transformées du courant oléfinique et introduire au moins une partie des paraffines non transformées, séparées du courant oléfinique dans l'unité de déshydrogénation.

24. La méthode de n'importe quelles des revendications 1 à 23, qui comprend en outre introduire au moins une partie du courant d'hydrocarbures alkyl aromatiques dans une unité de sulfonation, où l'unité de sulfonation est configurée pour sulfoner au moins une partie des hydrocarbures alkyl aromatiques du courant d'hydrocarbures alkyl aromatiques, de manière de produire des sulfonates alkyl aromatiques, où au moins une partie des sulfonates alkyl aromatiques produits consistent en sulfonates alkyl aromatiques ramifiés.
